(19) Europäisches Patentamt
European Patent Office
Office européen des brevets

(11) Veröffentlichungsnummer: **0 482 045 B1**

(12) **EUROPÄISCHE PATENTSCHRIFT**

(45) Veröffentlichungstag der Patentschrift: **22.02.95**

(51) Int. Cl.⁶: **D21C 5/02**

(21) Anmeldenummer: **90910688.2**

(22) Anmeldetag: **10.07.90**

(86) Internationale Anmeldenummer:
**PCT/EP90/01122**

(87) Internationale Veröffentlichungsnummer:
**WO 91/01405 (07.02.91 91/04)**

# (54) VERFAHREN ZUR ALTPAPIERAUFBEREITUNG.

(30) Priorität: **14.07.89 DE 3923393**

(43) Veröffentlichungstag der Anmeldung:
**29.04.92 Patentblatt 92/18**

(45) Bekanntmachung des Hinweises auf die Patenterteilung:
**22.02.95 Patentblatt 95/08**

(84) Benannte Vertragsstaaten:
**AT BE CH DE DK ES FR GB IT LI NL SE**

(56) Entgegenhaltungen:
**EP-A- 0 067 333**
**DE-A- 1 517 172**
**DE-A- 2 148 590**
**GB-A- 1 347 971**
**JP-A- 5 352 705**

(73) Patentinhaber: **Henkel Kommanditgesellschaft auf Aktien**

**D-40191 Düsseldorf (DE)**

(72) Erfinder: **Daute, Peter**
**Kreuzeskirchstrasse 5**
**W-4300 Essen 1 (DE)**
Erfinder: **Stoll, Gerhard**
**Danziger Strasse 69**
**W-4052 Korschenbroich 1 (DE)**
Erfinder: **Hornfeck, Klaus**
**August-Burberg-Strasse 34**
**W-4020 Mettmann (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Die Erfindung betrifft ein Verfahren zur Altpapieraufbereitung sowie die Verwendung von Alkoxylierungsprodukten bestimmter OH-gruppenhaltiger $C_{10-22}$-Carbonsäurederivate und/oder bestimmmter OH-gruppenhaltiger $C_{10-22}$-Carbonsäuren zur Altpapieraufbereitung.

Zur Herstellung von beispielsweise Zeitungdruck- und Hygienepapieren werden heute in großen Mengen Altpapiere eingesetzt. Für diese Papiersorten sind Helligkeit und Farbe Qualitätsmerkmale. Um diese zu erreichen, müssen die Druckfarben aus den bedruckten Altpapieren entfernt werden. Üblicherweise geschieht dies mittels Deinking-Verfahren, die im wesentlichen in zwei Teilschritten ablaufen:

1. Aufschlagen der Altpapiere, d. h. Zerfasern in Wasser bei gleichzeitigem Einwirken der für die Ablösung der Druckfarbenteilchen benötigten Chemikalien und
2. Ausscheidung der abgelösten Druckfarbenteilchen aus der Faserstoffsuspension.

Der zweite Verfahrensschritt kann durch Auswaschen oder Flotation erfolgen (Ullmanns Enzyklopädie der technischen Chemie, 4. Auflage, Band 17, Seiten 570 bis 571 (1979)). Bei der Flotation, bei der die unterschiedliche Benetzbarkeit von Druckfarben und Papierfasern ausgenutzt wird, wird Luft durch die Faserstoffsuspensionen gedrückt oder gesaugt. Dabei verbinden sich kleine Luftbläschen mit den Druckfarbenteilchen und bilden an der Wasseroberfläche einen Schaum, der mit Stoffängern entfernt wird.

Üblicherweise wird das Deinken von Altpapieren bei alkalischen pH-Werten in Gegenwart von Alkalihydroxiden, Alkalisilikaten, oxidativ wirkenden Bleichmitteln und oberflächenaktiven Substanzen bei Temperaturen zwischen 30 und 50 °C durchgeführt. Als oberflächenaktive Substanzen werden überwiegend Tenside anionischer und/oder nichtionischer Natur eingesetzt, beispielsweise Seifen, ethoxylierte Fettalkohole und/oder ethoxylierte Alkylphenole (Wochenblatt für Papierfabrikation <u>17</u>, 646 bis 649 (1985)).

Die DE-A-34 01 444 betrifft ein Verfahren zum Deinken von Altpapier unter Verwendung einer Verbindung der allgemeinen Formel

$$RCOO—(CH_2CH_2O)_m—(AO)_n—R'$$

in der R eine Alkyl- oder Alkenylgruppe mit 7 bis 21 C-Atomen, R' ein Wasserstoffatom oder eine Alkyl-, Alkenyl- oder Acylgruppe mit 1 bis 18 C-Atomen, AO $C_3H_6O$- oder $C_4H_8O$-gruppen oder eine Mischung aus $C_2H_4O$-, $C_3H_6O$- und $C_4H_8O$-gruppen, m eine ganze Zahl von 1 bis 100 und n eine ganze Zahl von 1 bis 100 bedeuten.

Die Verwendung von ethoxylierten Rizinusölen zum Deinken von bedruckten Altpapieren ist beispielsweise aus JP-A-78/52705, referiert in Chem. Abstr. 89, 131445j (1978) und DE-A-21 48 590 bekannt. In dem japanischen Schutzrecht werden Mischungen aus Rizinusöl mit 10 bis 400 Prozent Ethylenoxid und ethoxyliertem Nonylphenol beschrieben, die sich zur Entfernung von Druckfarben aus bedruckten Altpapieren eignen. Das in der DE-A-21 48 590 geschützte Verfahren betrifft organische Materialien, beispielsweise Papier, die mit Natriumchlorit in Gegenwart von organischen Verbindungen mit wenigstens einer Ethylenoxid-und/oder Propylenoxideinheit gebleicht werden. Als organische Verbindung mit wenigstens einer Alkylenoxideinheit eignet sich unter anderen ethoxyliertes Rizinusöl.

Bei Verwendung ethoxylierter Rizinusöle muß jedoch in Kauf genommen werden, daß die auf dem Markt erhältlichen Mengen an Rizinusöl und damit auch an ethoxylierten Rizinusölen starken Schwankungen unterworfen sind. Mißernten in den Hauptanbaugebieten Brasilien und Indien führen in mehr oder weniger großen Abständen zu einer Verknappung des Ausgangsmaterials Rizinusöl. Es besteht daher ein Bedürfnis nach einem Ersatz für ethoxylierte Rizinusöle, die, in Verfahren zur Altpapieraufbereitung eingesetzt, zumindest zu ebenso guten Deinkingergebnissen führen wie das zu ersetzende Produkt. Vor allem sollte das Austauschprodukt von einer breiteren, weniger krisenanfälligen Rohstoffbasis aus zugänglich sowie ökologisch und toxikologisch unbedenklich sein.

Es wurde nun gefunden, daß sich bestimmte OH-gruppenhaltige Carbonsäurederivate und/oder OH-gruppenhaltige Carbonsäuren, die mit Alkylenoxiden alkoxyliert sind, als Ersatz für ethoxylierte Rizinusöle in Verfahren zur Altpapieraufbereitung in hervorragender Weise eignen.

Erfindungsgegenstand ist dementsprechend ein Verfahren zur Altpapieraufbereitung durch bedruckte Altpapiere in Gegenwart von Alkoxylierungsprodukten aufzuschlagen und anschließend die abgelösten Druckfarbenteilchen durch Flotation oder Waschen aus den Papierstoffsuspensionen zu entfernen, welches dadurch gekennzeichnet ist, daß Alkoxylierungsprodukte, erhältlich durch Umsetzung von Alkylenoxiden mit $C_{10-22}$-Carbonsäurederivatenund/oder $C_{10-22}$-Carbonsäuren, die Carbonsäurereste mit wenigstens einer OH-Gruppe in Stellung 9, 10, 13 und/oder 14 enthalten, eingesetzt werden.

Ferner ist die Verwendung von Alkoxylierungsprodukten erhältlich durch Umsetzung von Alkylenoxiden mit $C_{10-22}$-Carbonsäurederivatenund/oder $C_{10-22}$-Carbonsäuren, die Carbonsäurereste mit wenigstens einer OH-Gruppe in Stellung 9, 10, 13 und/oder 14 enthalten, zur Altpapieraufbereitung Gegenstand der Erfindung.

Die erfindungsgemäß einzusetzenden Alkoxylierungsprodukte können nach gängigen organischen Synthesemethoden hergestellt werden. Als Ausgangsmaterialien für alkoxylierte OH-gruppenhaltige $C_{10-22}$-Carbonsäuren eignen sich alle OH-gruppenfreien, ungesättigten $C_{10-22}$-Carbonsäuren natürlichen und/oder synthetischen Ursprungs mit wenigstens einer oder zwei Doppelbindungen in 9- und/oder 13-Stellung, beispielsweise 9c-Dodecensäure, 9c-Tetradecensäure, 9c-Hexadecensäure, 9c-Octadecensäure, 9t-Octadecensäure, 9c, 12c-Octadecadiensäure, 9c, 12c, 15c-Octadecatriensäure, 9c-Eicosensäure und/oder 13c-Docosensäure und/oder Mischungen mit wenigstens einem hohen Gehalt solcher ungesättigten Carbonsäuren. Als Edukte werden vorzugsweise $C_{16-22}$-Carbonsäuren mit wenigstens einer oder zwei Doppelbindungen in 9- und/oder 13-Stellung oder Carbonsäuregemische mit wenigstens einem hohen Gehalt an $C_{16-22}$-Carbonsäuren, die wenigstens eine oder zwei Doppelbindungen in 9- und/oder 13-Stellung enthalten, eingesetzt.

Als Edukte für alkoxylierte OH-gruppenhaltige $C_{10-22}$-Carbonsäurederivate eignen sich alle OH-gruppenfreien, ungesättigten, natürlich vorkommenden und/oder synthetisch herstellbaren $C_{10-22}$-Carbonsäurederivate, die Carbonsäurereste mit wenigstens einer oder zwei Doppelbindungen in 9- und/oder 13-Stellung enthalten. Beispiele für ungesättigte Carbonsäurereste mit 10 bis 22 C-Atomen sind die bereits oben genannten Carbonsäuren. Ungesättigte Carbonsäurederivate, die $C_{16-22}$-Carbonsäurereste mit wenigstens einer oder zwei Doppelbindungen in 9- und/oder 13-Stellung enthalten, werden bevorzugt. Als ungesättigte $C_{10-22}$-Carbonsäurederivate eignen sich beispielsweise ungesättigte $C_{10-22}$-Carbonsäureester,ungesättigte $C_{10-22}$-Carbonsäureamide, ungesättigte $C_{10-22}$-Carbonsäuremono-und/oder -di-$C_{1-4}$-alkylamide und/oder ungesättigte $C_{10-22}$-Carbonsäuremono- und/oder -di-$C_{1-4}$-alkanolamide. Vorzugsweise werden ungesättigte $C_{10-22}$-Carbonsäurealkylester mit 1 bis 18 C-Atomen im einwertigen Alkoholrest und/oder Mono-, Di- und/oder Triglyceride, die $C_{10-22}$-Carbonsäurereste mit wenigstens einer oder zwei Doppelbindungen in 9- und/oder 13-Stellung enthalten, eingesetzt.

Beispiele für ungesättigte $C_{10-22}$-Carbonsäure-$C_{1-18}$-alkylester, die in an sich bekannter Weise durch Veresterung der entsprechenden ungesättigten Carbonsäuren oder durch Umesterung der entsprechenden Mono-, Di- und/oder Triglyceride mit $C_{1-18}$-Alkylalkoholen, beispielsweise Methanol, Ethanol, Propanal, Butanol, Isobutanol, 2-Ethylhexanol, Decanol und/oder Stearylalkohol, zugänglich sind, sind Palmitoleinsäuremethylester, Ölsäuremethylester, Ölsäureethylester, Ölsäureisobutylester, Ölsäure-2-ethylhexylester und/oder Ölsäuredecylester und/oder $C_{10-22}$-Carbonsäure-$C_{1-18}$-alkylestergemische mit wenigstens einem hohen Gehalt an solchen $C_{10-22}$-Carbonsäure-$C_{1-18}$-alkylestern, die in den Carbonsäureresten wenigstens eine oder zwei Doppelbindungen in 9- und/oder 13-Stellung haben, wie Palmölmethylester, Sojaölmethylester, Rübolmethylester und/oder Talgfettsäureethylester. Als Mono-, Di-und/oder Triglyceride, die OH-gruppenfreie ungesättigte $C_{10-22}$-Carbonsäurereste mit wenigstens einer oder zwei Doppelbindungen in 9- und/oder 13-Stellung enthalten, eignen sich insbesondere Fette und/oder Öle natürlichen Ursprungs, deren Carbonsäuregehalt sich überwiegend aus ungesättigten $C_{10-22}$-Carbonsäuren mit wenigstens einer oder zwei Doppelbindungen in 9- und/oder 13-Stellung, vorzugsweise überwiegend aus ungesättigten $C_{16-22}$-Carbonsäuren mit wenigstens einer oder zwei Doppelbindungen in 9- und/oder 13-Stellung zusammensetzt, wie Olivenöl, Leinöl, Sonnenblumenöl, Safloröl, Sojaöl, Erdnußöl, Baumwollsaatöl, erucasäurereiches und/oder erucasäurearmes Rüböl, Palmöl, Schmalz und/oder Talg.

Die ungesättigten $C_{10-22}$-Carbonsäurederivate und/oder ungesättigten $C_{10-22}$-Carbonsäuren werden beispielsweise nach dem in DE-PS 857 364 beschriebenen Verfahren durch Umsetzung mit Peressigsäure in Anwesenheit saurer Katalysatoren oder mit in situ aus Ameisensäure und Wasserstoffperoxid gebildeter Perameisensäure epoxidiert. Die Jodzahlen der erhaltenen Epoxidierungsprodukte liegen unterhalb 20, vorzugsweise unterhalb 15.

Anschließend werden die Oxiranringe der epoxidierten Carbonsäurederivate und/oder Carbonsäuren durch Umsetzung mit Wasserstoff oder protischen Verbindungen, wie Wasser, gerad- und/oder verzweigtkettigen $C_{1-18}$-Alkyl- und/oder $C_{2-18}$-Alkenylalkoholen oder gerad- und/oder verzweigtkettigen, gesättigten und/oder ungesättigten $C_{1-22}$-Carbonsäuren, unter Ausbildung von Hydroxygruppen aufgespalten. Die Bedingungen der Aufspaltung werden so gewählt, daß die vorhandenen Säurederivat- und Säuregruppierungen intakt bleiben.

Die Hydrierung von epoxidierten Carbonsäurederivaten und/oder epoxidierten Carbonsäuren kann beispielsweise analog dem in DE-A-20 21 530 beschriebenen Verfahren in Gegenwart von Katalysatoren auf Basis von Schwermetallen der VIII. Gruppe des Periodensystems bei Temperaturen zwischen 100 und 250 °C unter Wasserstoffdrücken zwischen $10^6$ und $5 \cdot 10^6$ Pa durchgeführt werden.

Die Umsetzungen epoxidierter Carbonsäurederivate und/oder epoxidierter Carbonsäuren mit protischen Verbindungen kann gemäß den in M. S. Malinovskii "Epoxides and their Derivatives", Sivon Press 1965 beschriebenen Verfahren bei Temperaturen zwischen 50 und 200 °C und Drücken zwischen $10^5$ und $10^6$ Pa durchgeführt werden. Die Aufspaltung der Oxiranringe mit gerad- und/oder verzweigtkettigen $C_{1-18}$-Alkyl- und/oder $C_{2-18}$-Alkenylalkoholen, vorzugsweise mit gerad- und/oder verzweigtkettigen $C_{1-6}$-Alkylalkoholen wird vorzugsweise in Gegenwart saurer Katalysatoren, wie Schwefelsäure und/oder p-Toluolsulfonsäure durchgeführt.

Die durch Aufspaltung der Oxiranringe erhältlichen Carbonsäurederivate und Carbonsäuren, die Carbonsäurereste mit wenigstens einer OH-Gruppe in 9-, 10-, 13- und/oder 14-Stellung enthalten, werden anschließend nach bekannten großtechnischen Verfahren mit vorzugsweise Ethylenoxid, Propylenoxid und/oder Butylenoxid, besonders bevorzugt mit Ethylenoxid und/oder Propylenoxid, gegebenenfalls in Gegenwart von Katalysatoren, beispielsweise Kaliumhydroxid und/oder Natriummethylat bei Temperaturen zwischen 110 und 200 °C, vorzugsweise zwischen 140 und 175 °C und bei Drücken zwischen $10^5$ und $2 \cdot 10^6$ Pa, vorzugsweise zwischen $3 \cdot 10^5$ und $5 \cdot 10^5$ Pa alkoxyliert (siehe beispielsweise in "Chemische Technologie", Band 7, Seiten 131 bis 132, Carl-Hanser-Verlag, München/Wien (1986)). Der Alkylenoxidgehalt der alkoxylierenden OH-gruppenhaltigen Carbonsäurederivate und/oder Carbonsäuren liegt zwischen 2 und 400 Gew.-%, vorzugsweise zwischen 40 und 200 Gew.-%, jeweils bezogen auf die nichtalkoxylierten Verbindungen.

Die erfindungsgemäß einzusetzenden Alkoxylierungsprodukte, erhältlich aus $C_{10-22}$-Carbonsäurederivaten und /oder $C_{10-22}$-Carbonsäuren, die Carbonsäurereste mit wenigstens einer OH-Gruppe in Stellung 9, 10, 13 und/oder 14 enthalten, werden Papierstoffsuspensionen vorzugsweise in Mengen von 0,02 bis 2 Gew.-%, besonders bevorzugt von 0,1 bis 0,8 Gew.-%, jeweils bezogen auf lufttrockenen Papierstoff zugesetzt. Lufttrockener Papierstoff bedeutet, daß sich im Papierstoff ein Gleichgewichtszustand an innerer Feuchte eingestellt hat. Dieser Gleichgewichtszustand hängt von der Temperatur und von der relativen Feuchte der Luft ab.

In vielen Fällen kann das Deinking-Ergebnis, d. h. die Entfernung von Druckfarben aus bedruckten Altpapieren gesteigert werden, wenn die erfindungsgemäß einzusetzenden Alkoxylierungsprodukte in Kombination mit beispielsweise $C_{10-22}$-Fettsäuren, wie Olinor$^R$4010, Olinor$^R$4020 und/oder Olinor$^R$DG40 (Hersteller aller Produkte: Henkel KGaA), ethoxylierten Alkylalkohole mit 6 bis 22 C-Atomen, ethoxylierten Alkylphenolen, Polymeren, wie Polyacrylamiden und/oder Polydimethylamionethylmethacrylsäure, und/oder Copolymeren, beschrieben beispielsweise in DE-A-38 39 479, eingesetzt werden. Die Gesamtmenge dieser fakultativen Bestandteile liegt zwischen 0,1 und 1 Gew.-%, bezogen auf lufttrockenen Papierstoff.

In Gegenwart von erfindungsgemäßen Alkoxylierungsprodukten lassen sich wasserverdünnbare und/oder lösungsmittelhaltige, vorzugsweise wasserverdünnbare Druckfarben, beispielsweise Zeitungsrotationsfarben, Buchdruckfarben, Offsetdruckfarben, Illustrationstiefdruckfarben, Flexodruckfarben, Laserdruckfarben und/oder Verpackungstiefdruckfarben aus bedruckten Altpapieren, beispielsweise Zeitungen, Illustrierten, Computerpapieren, Zeitschriften, Broschüren, Formularen, Telefonbüchern und/oder Katalogen entfernen. Die nach dem erfindungsgemäßen Verfahren deinkten Altpapiere zeichnen sich durch sehr hohe Weißgrade aus.

Bedruckte Altpapiere werden bei einer Stoffdichte beispielsweise zwischen 1 und 5 Gew.-% in einem Stofflöser in wäßriger Lösung, die typischerweise 0 bis 1,5 Gew.-% 100%iges Wasserstoffperoxid, 0 bis 2,5 Gew.-% 99gew.-%ige NaOH, 0 bis 4,0 Gew.-% Natronwasserglas, Feststoffgehalt 35 Gew.-% (37 bis 40 °Be), 0,02 bis 2 Gew.-% erfindungsgemäße alkoxylierte OH-gruppenhaltige Carbonsäurederivate und/oder Carbonsäuren und 0 bis 1 Gew.-% der oben genannten fakultativen Bestandteile - alle Gewichtsprozentangaben beziehen sich auf lufttrockenes Altpapier - enthält, bei Temperaturen zwischen 20 und 60 °C zerkleinert. Nach einer Verweilzeit zwischen 60 und 120 Minuten bei Temperaturen zwischen 20 und 60 °C werden die Papierstoffsuspensionen in Wasser eingerührt oder mit Wasser versetzt, so daß 0,6 bis 1,6 gew.-%ige Papierstoffsuspensionen erhalten werden. Anschließend werden die abgelösten Druckfarbenteilchen in an sich bekannter Weise durch Auswaschen oder durch Flotation aus den Papierstoffsuspensionen ausgeschieden (Ullmanns Enzyklopädie der technischen Chemie, 4. Auflage, Band 17, Seiten 570 bis 571 (1979)). Vorzugsweise wird in an sich bekannter Weise, beispielsweise in einer Denver-Flotationszelle flotiert.

## Beispiele

1. Herstellung von ethoxyliertem OH-gruppenhaltigem Sojaöl aus hydriertem Sojaölepoxid (Sojaöl - EO I)

In einem Autoklaven wurden 20 kg epoxidiertes Sojaöl (ungefähre Fettsäurezusammensetzung: 8 Gew.-% Palmitinsäure, 4 Gew.-% Stearinsäure, 28 Gew.-% Ölsäure, 53 Gew.-% Linolsäure und 6 Gew.-% Linolensäure; Epoxidgehalt = 6,78 Gew.-%; Jodzahl = 5; Säurezahl = 0,4) und 0,2 kg eines Nickelkatalysators (Trägermaterial: Kieselgur) vorgelegt, die im Reaktor vorhandene Luft durch Stickstoffspülung verdrängt und bei 150 bis 170 °C mit einem Wasserstoffdruck von 2 . $10^6$ Pa bis zum Ende der Wasserstoffaufnahme (ca. 6 Stunden) hydriert. Nach Abkühlen und Abtrennen des Katalysators wurden 20 kg farbloses hydriertes Sojaölepoxid mit einer OH-Zahl (OHZ) von 165,8, einer Verseifungszahl (VZ) von 181,4, einer Jodzahl (JZ) von 8,3 und einer Säurezahl (SZ) von 1 erhalten.

650 g des hydrierten Sojaölepoxids wurden mit 5,0 g einer 30 gew.-%igen Lösung von Kaliumhydroxid in Methanol versetzt und in einem Autoklaven auf 100 °C erhitzt. Bei dieser Temperatur wurden die vorhandenen Methanolspuren durch fünfmaliges Evakuieren und Belüften mit Stickstoff entfernt. Nach Erhöhung der Reaktionstemperatur auf 150 °C wurden insgesamt 308 g Ethylenoxid portionsweise zudosiert, so daß der Druck im Reaktor einen Wert von 5 . $10^5$ Pa nicht überstieg. Nach Beendigung der Reaktion wurde auf etwa 90 °C abgekühlt und zur Abtrennung noch vorhandener Ethylenoxidspuren ca. 15 Minuten evakuiert. Es wurde eine klare gelbe Flüssigkeit mit einer OHZ von 124,5 erhalten.

2. Herstellung von ethoxyliertem und propoxyliertem, OH-gruppenhaltigem Sojaöl aus hydriertem Sojaölepoxid (Sojaöl - EO/PO I)

371 g des gemäß Beispiel 1 hydrierten Sojaölepoxids wurden mit 6,2 g einer 30 gew.-%igen Lösung von Kaliumhydroxid in Methanol versetzt und unter den in Beispiel 1 angegebenen Bedingungen zuerst mit 440 g Ethylenoxid und anschließend im gleichen Reaktor mit 232 g Propylenoxid umgesetzt. Nach Entfernung der Propylenoxidspuren im Vakuum und Neutralisation des Katalysators mit 3,3 g Milchsäure wurde eine goldgelbe Flüssigkeit mit einer OHZ von 72,1 erhalten.

3. Herstellung von ethoxyliertem OH-gruppenhaltigem Leinöl aus hydriertem Leinölepoxid (Leinöl - EO I)

Gemäß Beispiel 1 wurden 1 200 g epoxidiertes Leinöl (ungefähre Fettsäurezusammensetzung: 5 Gew.-% Palmitinsäure, 4 Gew.-% Stearinsäure, 22 Gew.-% Ölsäure, 17 Gew.-% Linolsäure und 52 Gew.-% Linolensäure; Epoxidgehalt: 8,9 Gew.-%; Jodzahl = 10; Säurezahl = 0,7) und 15 g eines Nickelkatalysators (Trägermaterial: Kieselgur) in einem Autoklaven vorgelegt, die im Reaktor vorhandene Luft durch Stickstoffspülung verdrängt und bei 150 bis 170 °C mit einem Wasserstoffdruck von 2 . $10^6$ Pa bis zum Ende der Wasserstoffaufnahme hydriert. Nach Abkühlen und Abtrennen des Katalysators wurde farbloses hydriertes Leinölepoxid mit einer OHZ von 202,6, einer VZ von 178,2, einer JZ von 16,9 und einer SZ von 0,7 erhalten.

650 g des hydrierten Leinölepoxids wurden gemäß Beispiel 1 mit 308 g Ethylenoxid umgesetzt. Es wurde eine gelbe Flüssigkeit mit einer OHZ von 152 erhalten.

4. Herstellung von ethoxyliertem OH-gruppenhaltigem Sojaöl aus mit Methanol umgesetztem Sojaölepoxid (Sojaöl - EO II)

2360 g (10 Mol) epoxidiertes Sojaöl (Kenndaten analog Beispiel 1) wurden unter intensiver Kühlung zu der unter Rückfluß siedenden Lösung von 9 g (0,9 g/Mol Epoxid) konzentrierter Schwefelsäure in 960 g (30 Mol) Methanol getropft. Nach beendeter Epoxidzugabe und Abreaktion des Epoxids wurde das Reaktionsgemisch mit Diethylethanolamin neutralisiert und überschüssiges Methanol im Vakuum entfernt. Es wurde eine klargelbe Flüssigkeit mit einer OHZ von 165, einer VZ von 163, einer JZ von 19,4 und einer SZ von 1,6 erhalten.

510 g des Ringöffnungsproduktes von Sojaölepoxid mit Methanol wurden mit 7,5 g einer 30 gew.-%igen methanolischen Natriummethylatlösung versetzt und unter den in Beispiel 1 angegebenen Bedingungen bei 175 °C mit 551 g Ethylenoxid umgesetzt. Nach Entfernen der Ethylenoxidspuren im Vakuum wurde eine rotbraune Flüssigkeit mit einer OHZ von 110,6 erhalten.

5. Herstellung von ethoxyliertem und propoxyliertem OH-gruppenhaltigem Sojaöl aus mit Methanol umgesetztem Sojaölepoxid (Sojaöl - EO/PO II)

510 g des Ringöffnungsproduktes von Sojaölepoxid mit Methanol aus Beispiel 4 wurden mit 7,5 g einer 30 gew.-%igen methanolischen Natriummethylatlösung versetzt und unter den in Beispielen 1 und 2 angegebenen Bedingungen zuerst mit 551 g Ethylenoxid und anschließend im gleichen Reaktor mit 290 g Propylenoxid umgesetzt. Nach Entfernen der Propylenoxidspuren im Vakuum wurde eine braungelbe, fast klare Flüssigkeit mit einer OHZ von 94,9 erhalten.

6. Herstellung von ethoxyliertem OH-gruppenhaltigem Sojaöl aus mit Carbonsäuren umgesetztem Sojaölepoxid (Sojaöl - EO III)

In einem Rührkessel wurden 126 kg (805 Mol) einer Mischung gesättigter Fettsäuren (60 Gew.-% Octansäure, 35 Gew.-% Decansäure, 3 Gew.-% Dodecansäure und 2 Gew.-% Hexansäure; SZ = 361,9, Jz <1) und 180 kg (766 Mol) epoxidiertes Sojaöl (Kenndaten wie in Beispiel 1 angegeben) vorgelegt und unter Rühren auf 170 °C erwärmt. Nachdem das Reaktionsgemisch keine Epoxidgruppen mehr enthielt (ca. 4 Stunden) wurde im Vakuum bis etwa 190 °C destilliert. Es wurde eine dunkelgelbe Flüssigkeit mit einer OHZ von 84,6, einer VZ von 239 und einer SZ von 2,4 erhalten.

423 g des Umsetzungsproduktes von Sojaölepoxid mit Carbonsäuren wurden mit 6,9 g einer 30 gew.-%igen Lösung von Kaliumhydroxid in Methanol versetzt und analog Beispiel 1 bei 140 °C mit 660 g Ethylenoxid umgesetzt. Nach Entfernen der Ethylenoxidspuren im Vakuum und Neutralisation mit Milchsäure wurde eine dunkelgelbe Flüssigkeit mit einer OHZ von 54,7 erhalten.

Anwendungsbeispiel

100 g lutro (= 90 g atro bei 10 % Feuchte) bedrucktes Altpapier (lutro = lufttrocken, atro = absolut trocken) aus 50 Gew.-% Illustrierten und 50 Gew.-% Tageszeitungen wurden bei 3 Gew.-% Stoffdichte im Laborpulper mit einer wäßrigen Lösung, enthaltend
2 Gew.-% Natronwasserglas, 37 bis 40 °Be (35 gew.-%ig)
1 Gew.-% Natriumhydroxid (99 gew.-%ig)
0,7 Gew.-% Wasserstoffperoxid (100 gew.-%ig)
0,15 oder 0,4 Gew.-% erfindungsgemäße alkoxylierte OH-gruppenhaltige Verbindungen
bei 45 °C mittels Dispergierscheibe (3 500 Umdrehungen/Minute) 15 Minuten aufgeschlagen und nach 105 Minuten bei 45 °C auf 1 Gew.-% verdünnt, indem die Papierstoffsuspensionen in Wasser eingerührt wurden. Anschließend wurden die Papierstoffsuspensionen bei 45 °C in einer Denver-Flotationszelle bei 1 000 Umdrehungen/Minute innerhalb von 12 Minuten flotiert.

Die Deinking-Ergebnisse sind in Tabelle 1 zusammengefaßt. Die Deinkbarkeitsmaßzahl (DEM) wurde aus den Reflexionsfaktoren $R_{457nm}$ (Weißgrad) der bedruckten (BS), deinkten (DS) und unbedruckten (US) Papierstoffe nach folgender Formel errechnet:

$$DEM(\%) = \frac{\text{Weißgrad (DS)} - \text{Weißgrad (BS)}}{\text{Weißgrad (US)} - \text{Weißgrad (BS)}} \times 100$$

(0 % bedeutet keine Druckfarbenentfernung, 100 % bedeutet quantitative Druckfarbenentfernung).

Tabelle 1

| eingesetztes Alkoxylierungsprodukt | eingesetzte Menge an Alkylierungsprodukt in g pro 100 g lutro bedrucktem Altpapier | $R_{457}$ (BS) | $R_{457}$ (DS) | $R_{457}$ (US) | DEM (%) |
|---|---|---|---|---|---|
| Sojaöl - EO I | 0,4 | 39,7 | 56,2 | 61,4 | 76,0 |
| Leinöl - EO I | 0,4 | 35,2 | 54,1 | 60,2 | 75,6 |
| Sojaöl - EO/PO I | 0,15 | 40,2 | 57,1 | 61,7 | 78,6 |
| Sojaöl - EO II | 0,15 | 40,2 | 56,4 | 61,7 | 75,3 |
| Sojaöl - EO/PO II | 0,15 | 40,2 | 57,1 | 61,7 | 78,6 |
| Sojaöl - EO III | 0,15 | 40,2 | 57,6 | 61,7 | 80,9 |

## Patentansprüche

**1.** Verfahren zur Altpapieraufbereitung durch bedruckte Altpapiere in Gegenwart von Alkoxylierungsprodukten aufzuschlagen und anschließend die abgelösten Druckfarbenteilchen durch Flotation oder Waschen aus den Papierstoffsuspensionen zu entfernen, dadurch gekennzeichnet, daß Alkoxylierungsprodukte, erhältlich durch Umsetzung von Alkylenoxiden mit $C_{10-22}$-Carbonsäurederivaten und/oder $C_{10-22}$-Carbonsäuren, die Carbonsäurereste mit wenigstens einer OH-Gruppe in Stellung 9, 10, 13 und/oder 14 enthalten, eingesetzt werden.

**2.** Verfahren nach Anspruch 1, dadurch gekennzeichnet, daß bedruckte Altpapiere in Gegenwart von 0,02 bis 2 Gew.-%, vorzugsweise von 0,1 bis 0,8 Gew.-% Alkoxylierungsprodukten - jeweils bezogen auf lufttrockenen Papierstoff - aufgeschlagen werden.

**3.** Verfahren nach einem oder beiden der Ansprüche 1 bis 2, dadurch gekennzeichnet, daß zum Aufschlagen bedruckter Altpapiere solche Alkoxylierungsprodukte eingesetzt werden, die, bezogen auf nicht alkoxylierte Carbonsäurederivate und/oder nicht alkoxylierte Carbonsäuren, einen Alkylenoxidgehalt zwischen 2 und 400 Gew.-%, vorzugsweise zwischen 40 und 200 Gew.-% haben.

**4.** Verfahren nach einem oder mehreren der Ansprüche 1 bis 3, dadurch gekennzeichnet, daß zum Aufschlagen bedruckter Altpapiere ethoxylierte, propoxylierte und/oder butoxylierte, vorzugsweise ethoxylierte und/oder propoxylierte OH-gruppenhaltige $C_{10-22}$-Carbonsäurederivate und/oder $C_{10-22}$-Carbonsäuren eingesetzt werden.

**5.** Verfahren nach einem oder mehreren der Ansprüche 1 bis 4, dadurch gekennzeichnet, daß zum Aufschlagen bedruckter Altpapiere alkoxylierte OH-gruppenhaltige $C_{16-22}$-Carbonsäurederivate und/oder $C_{16-22}$-Carbonsäuren eingesetzt werden.

**6.** Verfahren nach einem oder mehreren der Ansprüche 1 bis 5, dadurch gekennzeichnet, daß zum Aufschlagen bedruckter Altpapiere alkoxylierte OH-gruppenhaltige Carbonsäurealkylester mit 1 bis 18 C-Atomen im einwertigen Alkoholrest und/oder alkoxylierte Mono-, Di- und/oder Triglyceride, die OH-gruppenhaltige Carbonsäurereste enthalten, eingesetzt werden.

**7.** Verwendung von Alkoxylierungsprodukten, erhältlich durch Umsetzung von Alkylenoxiden mit $C_{10-22}$-Carbonsäurederivaten und/oder $C_{10-22}$-Carbonsäuren, die Carbonsäurereste mit wenigstens einer OH-Gruppe in Stellung 9, 10, 13 und/oder 14 enthalten, zur Altpapieraufbereitung.

**8.** Verwendung nach Anspruch 7, dadurch gekennzeichnet, daß Alkoxylierungsprodukte mit Alkylenoxidgehalten zwischen 2 und 400 Gew.-%, vorzugsweise zwischen 40 und 200 Gew.-% - jeweils bezogen auf nicht alkoxylierte Carbonsäurederivate und/oder nicht alkoxylierte Carbonsäuren - verwendet werden.

**9.** Verwendung nach einem oder mehreren der Ansprüche 7 bis 8, dadurch gekennzeichnet, daß ethoxylierte, propoxylierte und/oder butoxylierte, vorzugsweise ethoxylierte und/oder propoxylierte OH-gruppenhaltige $C_{10-22}$-Carbonsäurederivate und/oder $C_{10-22}$-Carbonsäuren verwendet werden.

**10.** Verwendung nach einem oder mehreren der Ansprüche 7 bis 9, dadurch gekennzeichnet, daß alkoxylierte OH-gruppenhaltige $C_{16-22}$-Carbonsäurederivate und/oder $C_{16-22}$-Carbonsäuren verwendet werden.

**11.** Verwendung nach einem oder mehreren der Ansprüche 7 bis 10, dadurch gekennzeichnet, daß alkoxylierte OH-gruppenhaltige Carbonsäurealkylester mit 1 bis 18 C-Atomen im einwertigem Alkoholrest und/oder alkoxylierte Mono-, Di- und/oder Triglyceride, die OH-gruppenhaltige Carbonsäurereste enthalten, verwendet werden.

## Claims

**1.** A process for the regeneration of wastepaper, in which printed wastepaper is refined in the presence of alkoxylation products and the detached printing ink particles are subsequently removed from the paper stock suspensions by flotation or by washing characterized in that alkoxylation products obtainable by reaction of alkylene oxides with $C_{10-22}$ carboxylic acid derivatives and/or $C_{10-22}$ carboxylic acids containing carboxylic acid residues with at least one OH group in the 9, 10, 13 and/or 14 position are used.

**2.** A process as claimed in claim 1, characterized in that printed wastepaper is refined in the presence of 0.02 to 2% by weight and preferably 0.1 to 0.8% by weight alkoxylation products, based on air dry paper stock.

**3.** A process as claimed in one or both of claims 1 to 2, characterized in that alkoxylation products containing from 2 to 400% by weight and preferably from 40 to 200% by weight alkylene oxide, based on non-alkoxylated carboxylic acid derivatives and/or non-alkoxylated carboxylic acids, are used for refining printed wastepaper.

**4.** A process as claimed in one or more of claims 1 to 3, characterized in that ethoxylated, propoxylated and/or butoxylated, preferably ethoxylated and/or propoxylated, $C_{10-22}$ carboxylic acid derivatives and/or $C_{10-22}$ carboxylic acids containing OH groups are used for refining printed wastepaper.

**5.** A process as claimed in one or more of claims 1 to 4, characterized in that alkoxylated $C_{16-22}$ carboxylic acid derivatives and/or $C_{16-22}$ carboxylic acids containing OH groups are used for refining printed wastepaper.

**6.** A process as claimed in one or more of claims 1 to 5, characterized in that alkoxylated OH-containing carboxylic acid alkyl esters containing 1 to 18 carbon atoms in the monohydric alcohol component and/or alkoxylated mono-, di- and/or triglycerides containing OH-containing carboxylic acid residues are used for refining printed wastepaper.

**7.** The use of alkoxylation products obtainable by reaction of alkylene oxides with $C_{10-22}$ carboxylic acid derivatives and/or $C_{10-22}$ carboxylic acids containing carboxylic acid residues with at least one OH group in the 9, 10, 13 and/or 14 position for the regeneration of wastepaper.

**8.** The use claimed in claim 7, characterized in that alkoxylation products containing from 2 to 400% by weight and preferably from 40 to 200% by weight alkylene oxide, based on non-alkoxylated carboxylic acid derivatives and/or non-alkoxylated carboxylic acids, are used.

**9.** The use claimed in one or more of claims 7 to 8, characterized in that ethoxylated, propoxylated and/or butoxylated, preferably ethoxylated and/or propoxylated, $C_{10-22}$ carboxylic acid derivatives and/or $C_{10-22}$ carboxylic acids containing OH groups are used.

**10.** The use claimed in one or more of claims 7 to 9, characterized in that alkoxylated $C_{16-22}$ carboxylic acid derivatives and/or $C_{16-22}$ carboxylic acids containing OH groups are used.

**11.** The use claimed in one or more of claims 7 to 10, characterized in that alkoxylated OH-containing carboxylic acid alkyl esters containing 1 to 18 carbon atoms in the monohydric alcohol component and/or alkoxylated mono-, di- and/or triglycerides containing OH-bearing carboxylic acid residues are

used.

## Revendications

1. Procédé de traitement des vieux papiers par broyage de vieux papiers imprimés en présence de produits d'alcoxylation et ensuite élimination des particules d'encre d'imprimerie détachées par flottation ou lavage à partir des suspensions de pâte à papier, caractérisé en ce qu'on met en oeuvre des produits d'alcoxylation, obtenues par réaction d'oxydes d'alkylène avec des dérivés d'acide carboxylique en $C_{10-22}$ et/ou d' acides carboxyliques en $C_{10-22}$, qui contiennent des radicaux d'acide carboxylique ayant au moins un groupe OH en position 9, 10, 13 et/ou 14.

2. Procédé selon la revendication 1, caractérisé en ce que des vieux papiers imprimés sont broyés en présence de 0,02 à 2 % en poids, de préférence de 0,1 à 0,8 % en poids de produits d'alcoxylation, rapportés respectivement à la pâte à papier séchée à l'air.

3. Procédé selon une ou les deux revendications 1 à 2, caractérisé en ce que pour broyer les vieux papiers imprimés on met en oeuvre des produits d'alcoxylation, qui par rapport aux dérivés d'acides carboxyliques non éthoxylés et/ou aux acides carboxyliques non alcoxylés ont une teneur en oxyde d'alkylène comprise entre 2 et 400 % en poids, de préférence entre 40 et 200 % en poids.

4. Procédé selon une ou plusieurs des revendications 1 à 3, caractérisé en ce que pour broyer des vieux papiers imprimés on met en oeuvre des dérivés d'acides carboxyliques en $C_{10-22}$ et/ou des acides carboxyliques en $C_{10-22}$ éthoxylés, propoxylés et/ou butoxylés, de préférence éthoxylés et/ou propoxylés contenant des groupes OH.

5. Procédé selon une ou plusieurs des revendications 1 à 4, caractérisé en ce que pour broyer des vieux papiers imprimés on utilise des dérivés d'acide carboxyliques en $C_{18-22}$ et/ou des acides carboxyliques en $C_{12-22}$ alcoxylés contenant des groupes OH.

6. Procédé selon une ou plusieurs des revendications 1 à 5, caractérisé en ce que pour broyer des vieux papiers imprimés on met en oeuvre des esters d'acides carboxyliques alcoxylés contenant des groupes OH ayant de 1 à 18 atomes de C dans le radical alcool monovalent et/ou des mono, di et/ou triglycérides, qui contiennent des radicaux d'acides carboxyliques contenant des groupes OH.

7. Utilisation pour le traitement des vieux papiers de produits d'alcoxylation, pouvant être obtenus par réaction d'oxydes d'alkylène avec des dérivés d'acides carboxyliques en $C_{10-22}$ et/ou des acides carboxyliques en $C_{10-22}$, qui contiennent des radicaux d'acides carboxyliques avec au moins un groupe OH en position 9, 10, 13 et/ou 14.

8. Utilisation selon la revendication 7, caractérisée en ce qu'on utilise des produits d'alcoxylation ayant des teneurs en oxyde d'alkylène comprises entre 2 et 400 % en poids, de préférence entre 40 et 200 % en poids, respectivement rapportés aux dérivés d'acides carboxyliques non alcoxylés et/ou aux acides carboxyliques non alcoxylés.

9. Utilisation selon une ou plusieurs des revendications 7 à 8, caractérisée en ce qu'on utilise des dérivés d'acides carboxyliques en $C_{10-22}$ et/ou des acides carboxyliques en $C_{10-22}$ éthoxylés, propoxylés et/ou butoxylés, de préférence éthoxylés et/ou propoxylés contenant des groupes OH.

10. Utilisation selon une ou plusieurs des revendications 7 à 9, caractérisée en ce qu'on utilise des dérivés d'acides carboxyliques en $C_{16-22}$ et/ou des acides carboxyliques en $C_{16-22}$ alcoxylés contenant de groupes OH.

11. Utilisation selon une ou plusieurs des revendications 7 à 10, caractérisée en ce qu'on utilise des esters d'alkyle d'acides carboxyliques avec de 1 à 18 atomes de C, alcoxylés contenant des groupes OH dans le radical alcool et/ou des mono, di et/ou triglycérides, qui contiennent des radicaux d'acides carboxyliques contenant des groupes OH.